**Europäisches Patentamt**

**European Patent Office**

**Office européen des brevets**

(11) Publication number : **0 482 858 A2**

# EUROPEAN PATENT APPLICATION

(21) Application number : **91309698.8**

(22) Date of filing : **21.10.91**

(51) Int. Cl.⁵ : **A61M 5/152,** A61F 7/10, A61F 7/00

(30) Priority : **26.10.90 US 604042**

(43) Date of publication of application :
**29.04.92 Bulletin 92/18**

(84) Designated Contracting States :
**AT BE CH DE DK ES FR GB GR IT LI LU NL SE**

(71) Applicant : **ALCON SURGICAL, INC.,**
**6201 South Freeway**
**Forth Worth, Texas 76134-2099 (US)**

(72) Inventor : **Tyler, Hugh Jean**
**12342 Red Hill Avenue**
**Santa Anna, California 92705 (US)**
Inventor : **Domash, David M.**
**23551 Via Calzada Way**
**Mission Viejo, California 92691 (US)**

(74) Representative : **Price, Nigel John King et al**
**A.A. Thornton & Co. Northumberland House**
**303-306 High Holborn**
**London WC1V 7LE (GB)**

(54) **Surgical infusion control system.**

(57)    An infusion system which is a single integrated unit and performs all fluid handling functions associated with controlled pressure is disclosed. The system comprises a multi-channeled bag (22), capable of carrying infusion fluid to a plurality of surgical tools, which rests between an upper plate (26) and a lower plate (28) is disclosed. The bag is sealed on one side by a header (34) which contains parts for receiving infusion or tool channels, and has attachment capabilities for receiving infusion fluids and gasses or tools. The upper plate includes on/off valves (48) in communication with the bag to control fluid flow path. This upper plate (26) also include a proportional valves (40), electronically corrected to a pressure sensors (44,45), forming a feedback system, in communication with the bag and capable of adjusting the infusion fluid pressure upon sensing external pressure changes. The lower plate (28) provides support and heat exchange to the bag, as it can be heated or cooled so as to heat or cool the infusion fluid. All of these functions of the system are preformed non-invasively whereby the infusion fluid remains sterile.

FIG. A

EP 0 482 858 A2

## Field of the Invention

The invention relates generally to surgical infusion systems, and more particularly to a device for allowing infusion of treatment fluids during surgery.

## Background Of The Invention

Surgery, particularly that involving invasion of a body cavity, sometimes requires infused fluid to replace that fluid which has been lost or to maintain pressure in the body cavity. Specifically, in ophthalmic surgery, controlled fluid infusion has many applications. For example, vitreous surgery requires fluid infusion into the ocular cavity to replace vitreous (removed by suction) and to sufficiently pressurize the ocular cavity, to maintain its shape.

Present methods of controlled infusion either involve gravity, in raising or lowering a bottle of infusion fluid, or a pressurized bottle of infusion fluid for injection into the body cavity. This is usually done with a bottle attached to various tubing. If the surgeon desires to cool or heat the infusion fluid, the tubing is run through a cold or hot water bath. These fluid paths in the tubing are not open to measure pressure, change pressure, or reduce flow. Also, additional gaseous or liquid components cannot be added to this infusion fluid unless the tubing connections are broken and the material is injected manually.

These present methods do not contemplate that a single apparatus, containing all components necessary to perform all of the functions associated with infusion, such as heat exchanging (heating or cooling), pressurizing, and pressure sensing, could be used. Rather, these functions are performed by separate components.

Were one to substitute a flexible fluid vessel, such as a plastic bag, for a pressurized bottle in order to facilitate heat exchange, additional components would be required to perform the additional functions necessary for carrying out the infusion procedure. Tubes extending from the bag would be required for connection to the components necessary for these other functions. The infusion fluid would be pressurized by separate units such as peristaltic pumps in conjunction with silicone tubing. Pressure sensing would be performed invasively by hydrophobic filters placed directly in the fluid flow path. There would then be the risk that these hydrophobic filters, by mere exposure to the infusion fluid, would contaminate the infusion fluid.

Because present infusion units often comprise multiple separate components, they have to be kept a far distance from the patient, and therefore outside the sterile field (the specific operating area). Accordingly, long tubes would be required to deliver infusion fluids from these components to the patient. These long tubes give rise to potential surgical errors, since the large volume of fluid present in these tubes inherently creates unacceptably long delays in controlling or terminating the flow of the infusion fluid. Also, these tubes can kink, tangle or fold over each other, causing the fluid flow to unintentionally and unexpectedly be reduced or cease.

These infusion devices also must be loaded with infusion fluid by someone other than the surgeon. Thus, the potential for error by infusing the incorrect fluid exists.

These prior art infusion systems do not allow for convenient tool interchangeability. Rather, the surgeon must manually turn stopcocks on tubing lines to switch between tools. This switching is time consuming, whereby it leads to potential surgical errors.

## Summary of the Invention

It is an object of this invention to provide an infusion system which performs all functions associated with infusion within a single integrated unit.

It is another object of this invention to provide a fluid infusion system where the fluid pressure within the system is noninvasively regulated, maintaining the sterility of the fluid.

It is a further object of the present invention to provide an apparatus which can sense pressure changes through a feedback system and proportionally adjust the pressure to compensate for these pressure changes.

It is yet another object to provide an infusion system which allows the use of multiple tools and permits simplified switching between tools.

A still further object of this invention to provide multiple infusion ports whereby additional gaseous components can be added to the infusion fluid noninvasively.

Yet another object of the present invention is to provide a small, lightweight infusion system which may be placed in the sterile field and controlled by the surgeon, thus minimizing the potential of any errors.

Another object of this invention to provide an infusion bag which is economical to manufacture and whose disposability makes it cost-effective to use.

It is still a further object to provide servo control of infusion fluid pressure.

The foregoing and other objects are accomplished by the illustrated embodiment of the invention described hereinafter which provides an infusion apparatus with all fluid handling and control components integrated into a single unit. The single unit provides controlled pressure infusion in response to sensing of system pressure. The apparatus includes pressure sensing means which allow it to sense pressure changes within the body cavity, and to adjust infusion pressure to accommodate for such pressure changes.

The apparatus includes as a fluid vessel a disposable bag which lies between an upper plate and a lower plate and which has formed therein an array of fluid channels. The bag is disposed to cooperate with components in these plates. The upper plate contains components including pressure sensors and proportional valves which, in combination with electronic circuitry, form a servo pressure control system. The upper plate additionally includes on/off valves. The lower plate provides support and heat exchange capability.

The bag itself contains several series of channels. These channels originate or terminate in ports located on a header, external to the bag. The bag includes a first transport channel which receives pre-pressurized fluid through a first inlet port on the header.

This first transport channel then becomes serpentine. This serpentine region terminates and splits into two channels. Both of these channels cooperate with the servo pressure control system (proportional valve and pressure sensors) on the upper plate. The first of these two channels splits into a first series of three end-branch channels while the second splits into a second series of two end branch channels. Each of the end branch channels cooperates with an on/off valve on the upper plate to control fluid flow therein. All of the channels either originate or terminate in ports located on a header. Port openings on the header allow for connections either to infusion fluid sources or to tools. Because both series of channels may supply pressure-regulated infusion fluid, as explained below, tools may be used with both.

The bag channels cooperate non-invasively with the components on the upper and lower plate. The unique arrangement of channels and plate components provides a single integrated controlled fluid infusion system capable of performing multiple fluid handling functions associated with controlled infusion, including heat exchange or temperature control, fluid flow path control, multiple tool selection and tool switching, continuous fluid pressure control and the mixing or dissolving of other gasses with the infusion fluid. All of this is accomplished without direct contact between the fluid and the control components.

Heat exchange or temperature control is performed in the serpentine channel region. The serpentine channel is in contact with the lower plate which may be heated or chilled by an external source. Since the fluid runs in a serpentine path over a heated or chilled region of the lower plate, heat is exchanged as the fluid within the channel contacts this plate for a time period sufficient to allow heating or cooling of the fluid.

Fluid flow path control is achieved by on/off valves mounted in the upper plate above the end branch channels. When activated, an on/off valve will close the respective end branch channel with which it cooperates. And when deactivated, the valve will open the respective and branch channel. Since only one on/off valve may be open for each series of end branch channels, the flow to each port on the header is thereby controlled.

This controlled fluid flow path allows for simultaneous connection of multiple tools and tool switching. The first series of end branch channels terminates in three tool ports on the header. Tool selection and switching may be accomplished occurs among upwards of three tools in the illustrative embodiment of the invention, depending on which on/off valve is deactivated. When a different on/off valve is deactivated, the previously activated on/off valve is activated and tool switching then occurs between the associated tools. Thus, the need for disconnecting tools prior to switching has been eliminated. Moreover, infusion can continue instantaneously upon tool switching. Also, the system is capable of using two tools simultaneously, as the second series of end branch channels also terminates in a tool port on the header. This end branch channel is also controlled by its on/off valve.

Fluid infusion pressure into operating tools can be controlled by this system. The on/off valve, associated with a tool, when activated, opens a flow channel leading to that tool. a pressure sensor, common to the entire series of end branch channels, senses the downstream pressure in the body and provides a pressure signal to the servo pressure control which operates a proportional valve located slightly upstream to maintain desired pressure. The pressure sensor is in electric communication with the proportional valves. This proportional valve then changes its pinch in response to a feedback signal from the pressure sensor, accommodating for any change in pressure in the body cavity. If a new flow path is opened, i.e., changing to a new tool, this pressure sensor will respond to this new downstream pressure in the body cavity and will cause the proportional valves to adjust the flow of infusion fluid, and thereby pressure, in the new tool accordingly. This adjustment is almost instantaneous whereby infusion pressure remains in proportion to that in the body cavity.

Finally, air or other gases may be infused into tools through an air/gas inlet port. Air or gas must leave through the end branch channel connected to the tool port as the air or gas enters the system at an end branch channel, downstream from the pressure sensor and the proportional valves.

Brief Description of the Drawings

FIG. 1 is a cut away top view of an embodiment of infusion control device in accordance with the present invention.

FIG. 2 is a side elevation view of the infusion control device of Fig 1.

FIG. 3 is a opposite side elevation view of the infusion control device of Fig 1.

FIG. 4 is a top view of the infusion control device of Fig 1.

Detailed Description

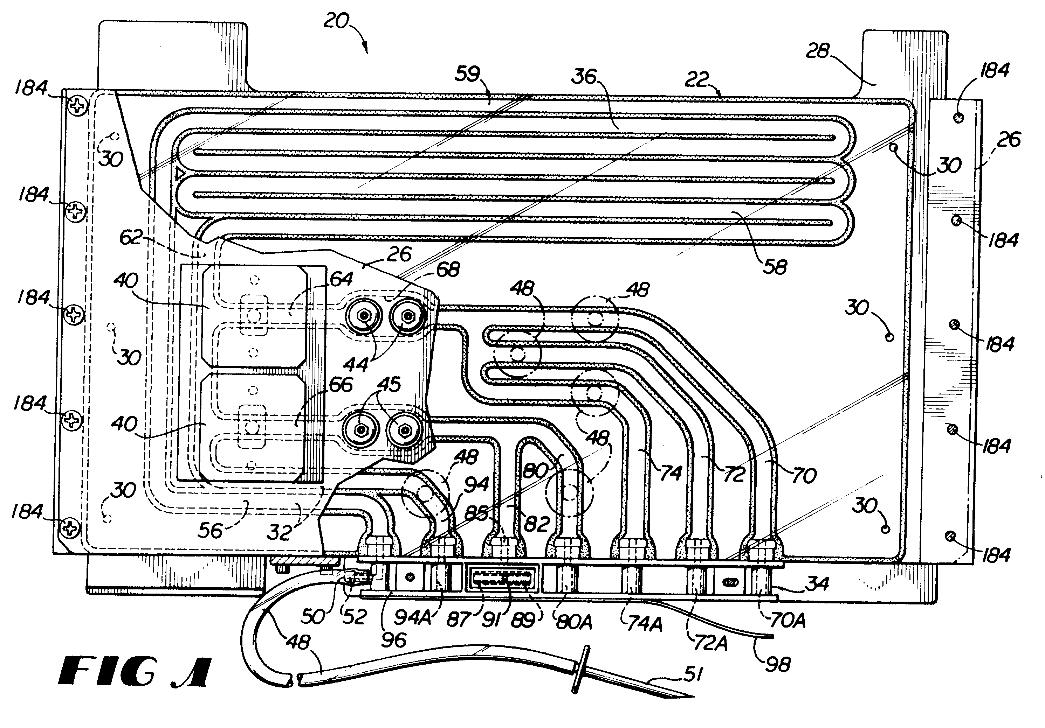

Turning now to the drawings, wherein like components are designated by like reference numerals throughout the various drawing figures, attention is first directed to FIG. 1, the preferred infusion control device of the system. This device includes an infusion bag 22 positioned in a small fixed gap between an upper metal plate 26 and a lower metal plate 28 (FIG. 4). While metal is preferred, any other sturdy material with good thermal conductivity and heat retention may be used.

The bag 22 is capable of performing fluidic control functions requiring very precise geometry, yet it has no rigid frame. The bag structure relies upon precise registration with the upper and lower metal plates 26, 28 which form a permanent rigid frame. The arrangement of the bag within the plates allows fixed geometry of sensors and actuators for precise proportional valve action and pressure control.

The infusion bag itself is made of two thin plastic sheets. The bag is manufactured by sealing the sheets, inflating the now formed bag and then heat setting the bag along a predefined pattern to form the channels. Preferably, the plastic sheets are polyolefins, including blends of polyethylene and ethylene vinyl acetate (EVA). The material is chosen such that it will function over a wide temperature range. Specifically, this material allows fluid flow in the bag channels with proportional fluid control when the bag contains very cold fluid. Additionally, this material permits fluid flow in the channels at very low pressures.

The registration holes 30 for engagement with corresponding registration pins on lower plate 28 are formed during the manufacturing process of the bag 22, whereby the bag 22 may be properly aligned when placed on plates 26, 28 of the mechanism. Overall, the manufacturing cost is low, whereby an economical disposable bag is provided.

The bag 22 contains various channels 32. These channels 32 either originate or terminate in a header 34. Along the fluid pathway formed by these channels 72 are a heat exchanger region 36 of lower plate 28, servo valves 40, pressure sensors 44, 45 and on/off valves 48.

A source of infusion fluid is introduced to the bag 22 through a tube 49 connected at an inlet fitting 50. The source of infusion fluid 49 is preferrably a bottle or other similar container of pressurized fluid, preferrably pressurized to 4 psig. This pressure is preferred since most infusion occurs at pressure well below 4 psig or 250 mmHg. Typically, requestec surgical pressures are 50-100 mmHg. However, even an unregulated infusion pressure of 250 mmHg will not damage the ocular cavity.

The inlet fitting 50 is attached to a tube 49 having a spike 51 for attachment to bottles or tubes containing pressurized fluid. The preferred spike is a vented spike which is suitable for attaching to tubing of a container of fluid pressurized to 4 psig. The spike 51 extends upward to permit fluid, but not air, to enter. The inlet fitting 50 communicates with an inlet port 52 on the header 34 and then with the transport channel 56. The transport channel 56 communicates with a series of serpentine-like channels 58 forming the heat exchange section 59. The heat exchange section 59 is capable of heating or cooling the fluid. In the preferred embodiment, the lower plate 28 (FIG. 4) is cooled either manually or by refrigeration. A conventional refrigeration system (not shown) includes a separate series of thin channels in lower plate 28 which have chilled fluid passed through them. The fluid may thus be chilled to the desired temperature and maintained at this desired temperature. In ophthalmalic surgery, cooling is preferred. A serpentine-like channel arrangement is preferred to ensure maximum heat exchange since the infusion fluid will be exposed to heating or cooling for the maximum possible time. Other suitable channel arrangements may also be used in the heat exchange section 59. However, the serpentine arrangement is optimal because it permits optimal fluid channel surface area contact with the lower plate 28.

The serpentine channels 58 terminate in a single flow channel 62 which splits into a first flow channel 64 and second flow channel 66. Each flow channel 64, 66 cooperates with a proportional valve 40, under servo control from and interfaced with pressure sensors 44. These flow channels are widened 68 at the points of cooperation or contact with the pressure sensors 44, 45. The first flow channel 64 splits into a first series of three end branch channels 70, 72, 74 with an on/off valve 48 cooperating with each end branch channel 70, 72, 74. The three end branch channels 70, 72, 74 terminate in the header 34, preferably in the three outermost ports 70A, 72A, 74A, for connection with various surgical tools. The second flow channel 68 separates into a second series of two end branch channels 80, 82 between the pressure sensor 45 and the header 34. The first end branch channel 80 is controlled by an on/off valve 48, and terminates in a tool port 80A in the header 34.

The second end branch channel 82 is open and terminates in a gas/air inlet port 85 on the header 34. The gas/air inlet port 85 is covered by a filter media 87, preferably a hydrophobic filter material. Header ribs 89 underneath this filter 87 prevent the filter 87 from collapsing and closing the gas/air opening 91. Gas is infused into this opening 91 through the hydrophobic filter 87. The preferred filter has .45 micron pores as this is the largest pore size which will sterilize air and gas. However, filters with smaller pore sizes are also sufficient. The infused gas then moves upstream through the second end branch channel 82 whereby the fluid in the first end branch channel 80 is displaced. Subsequently, this gas flows through the first end branch channel 80 and out through the corresponding port 80A. The gas/air inlet port 85 is used at the end of the surgical procedure, after all of the necessary vitreous has been removed. The infused gas fills the ocular cavity in place of the previously infused fluid (usually a balanced saline solution) to take tension caused by the infusion fluid off the retina and thereby enhance healing.

The portion of the flow channel 62 which extends beyond the branch off points for the first flow channel 64 and the second flow channel 65 forms an output channel 94. This channel 94 originates in a tool port 94A and communicates with an on/off valve 48. This output channel is not proportionally controlled. Rather, it is controlled only by the on/off valve 48. This output channel 94 supplies chilled infusion fluid (preferably at 4 psig) to a contact lens external to the eye so as to provide a moist interface between the lens and the eye, thereby providing optical coupling. This direct line to the eye is part of a separate loop, whereby there is no possibility that the fluid supplied to the other tools operating on the eye may become contaminated.

All of the channels are completely enclosed along their lengths. The fluid flow path is undisturbed except for points of interaction with the proportional valves 40, pressure sensors 44, 45 and on/off valves 48 which cooperate with the fluid flow path, but all these are external to the heat sealed plastic channels of the bag 22. As a result, the infusion system provides completely non-invasive proportional pinching, pressure sensing, pressure control, and infusion fluid flow control. Such a non-invasive system ensures sterility of the infusion fluid, since it remains untouched by instrumentation which could carry contaminating agents.

The number of fluid channels, and accordingly the number of pressure control loops, is a matter of design choice and is not critical to the practice of this invention. The two channels described here merely illustrate the function and structure of the apparatus of the invention.

The channels within the bag 22 either originate or terminate in a header 34. This header 34 is preferably of one-piece construction and encloses one side of the bag 22. The header 34 is designed such that it alone contains multiple input and output ports 52, 70a, 72A, 74A, 80a, 85A, 94A. These input and output ports include infusion supply port 52, a gas/air exchange port 85, and tool opening ports 70A, 72A, 74A, 80A, 94A. Since multiple tool opening ports are provided, the infusion control system can accommodate multiple tools and, in cooperation with the on/off valves 48, switch infusion fluid between them in an uninterrupted, non-invasive manner. The header 34 terminates in a cross member 96 which contains the port openings adapted to receive tools, tubing or the like.

Prior to tube and tool attachment, this header cross member is sealed with a sterile barrier such as Tyvek® 98, a sterilizable adhesive backed nonwoven material. Tyvek® is used in this preferred embodiment since it is a gas permeable polyolefin. This gas permeability allows the ports to be sterilized eliminating the need for sterile plugs in the port openings. Alternately, other methods to maintain a sterile fluid path may be used.

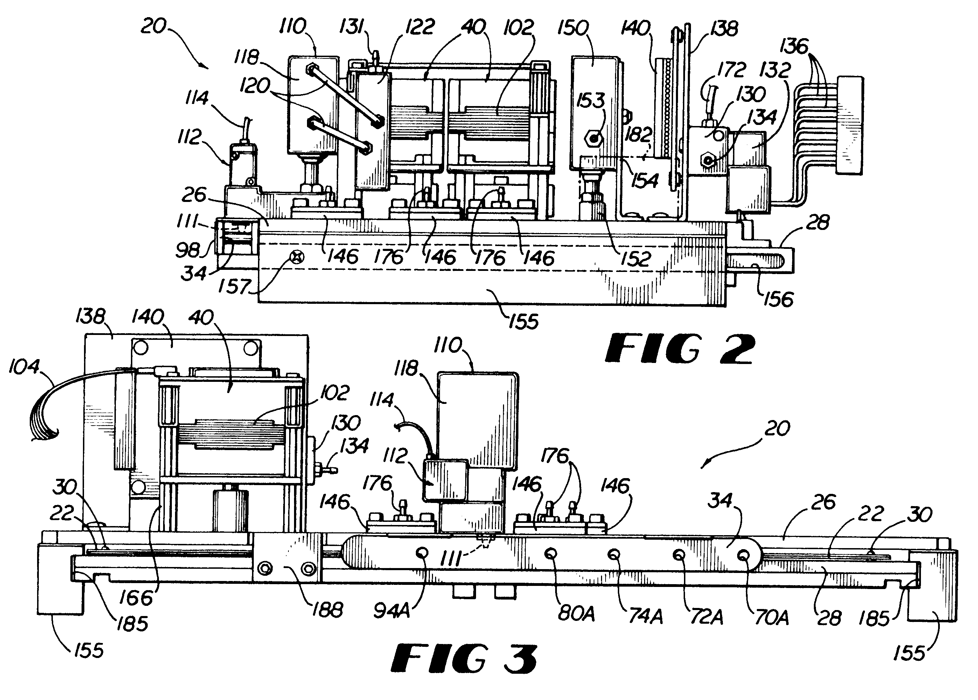

Turning now to FIG. 2, a side view of the device 20 is shown. The upper plate 26 contains several components which operate on the bag 22. These components include proportional (servo) valves 40, an air infusion mechanism 110, an actuator manifold 130, an electronic circuit board 140, on/off valves 146 and failsafe valves 150.

The servo valves 40 control proportional pinching of channels 32 in the bag 22 (FIG. 1). These servo valves 40 include stepper motors which drive finely threaded rods up and down according to the direction of rotation of the motors. Preferably, the rods are tipped with rubber or other soft material to avoid puncturing the bag. Fluid pressure in the channels is regulated by the extent to which the rod pinches the bag against the lower plate 28. The stepper motors 102 communicate through electrical lines 104 with pressure sensors (FIG. 4), which control the proportional pinching.

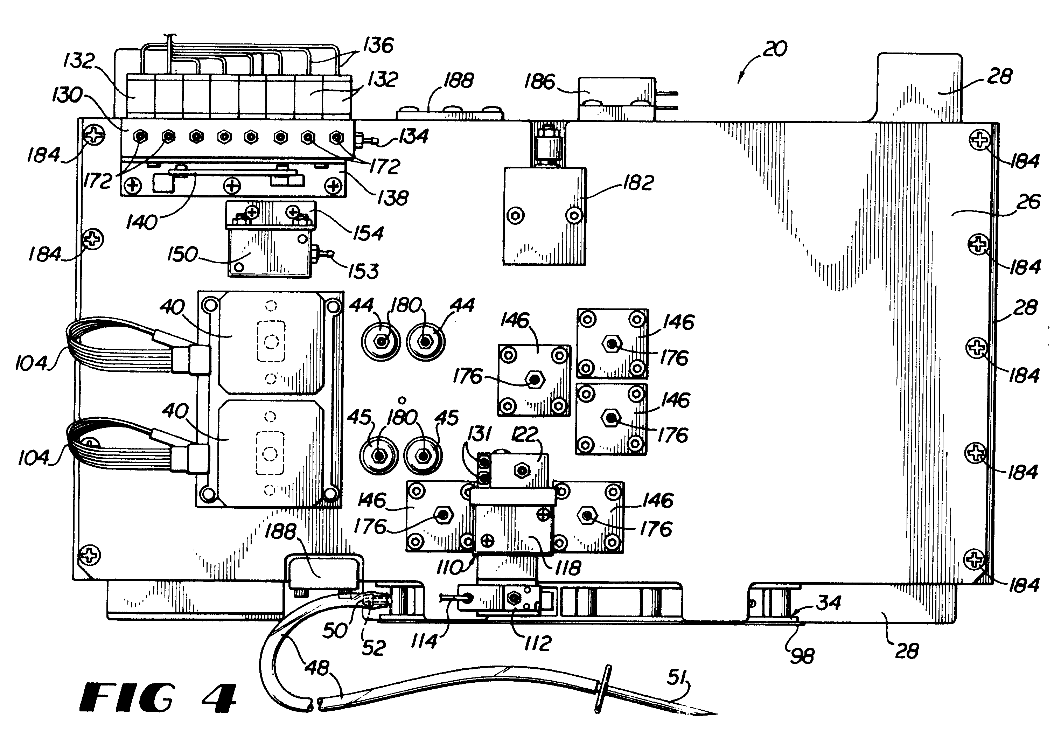

The air infusion mechanism 110 includes an air fitting 111 which is connected to a control valve 112. Pressurized air or gas from an outside source enters this control valve through a tube 114 (FIG. 3), whereby its flow through the air fitting 111 is regulated. When the activated air fitting 111 contacts the lid of the gas/air infusion inlet 85 on the header 34, gas is infused into the bag 22 (FIG. 1). As described above, this infused air then moves to the surgical site. Preferably, the gas is infused at about 4 psig, so as not to harm the ocular cavity if the valves do not function properly.

An air cylinder 118, attached by air tubes 120 to a pilot valve 122, communicates with the air fitting 111 moving it up or down when actuated. The pilot valve 122, depending on internal air pressure, moves the air cylinder 118 either up or down into the active infusion position. The pilot valve 122 is supplied with air from the actuator manifold 130 through pilot valve fittings 131.

The actuator manifold 130 is an air intake device containing several valves 132, preferably eight, which control pressurized air flow to the on/off valves 146 and the failsafe valve 150. This manifold 130 is supplied by an outside air source (not shown) pressurized to about 45 psig. This outside air source is connected to a fitting 134, preferably at the end of the manifold 130. The manifold valves 132 (FIG. 4) are powered via electrical lines 136 which are controlled by the system's software within its logic control unit (not shown) which is of conventional design.

The manifold 130 attaches to a bracket 138 anchored on the upper plate 26. This same bracket supports an electronic circuit board 140 of conventional design used in performing the control functions of the infusion system.

The on/off valves 146 are regulated by the air pressure, which is controlled by a valve 132 on the actuator manifold 130. The valves 146 are preferably silicone rubber diaphrams (not shown) which, when actuated by air pressure cause it to pinch the bag channel 32 (FIG. 1). However, any other suitable resilient elastic material may be used. When the pressure on the diaphram is released, the diaphram moves up and the material forming the bag channels is sufficiently resilient to return to its original state.

The actuator manifold 130 also communicates with a failsafe valve 150. The failsafe valve 150 is a safety device, which operates on logic opposite that of the on/off valves 146 controlled by the actuator manifold valves 132. That is, if the failsafe valve 152 does not receive pressurized air or power from the manifold through the failsafe valve air opening 153, its spring biased valve will activate and shut off the fluid pressure in the entire system, which may be infusing fluid at 4 psig. This failsafe valve is attached to the upper plate by a bracket 154, but any other equivalent fastening means may be used.

This figure also shows the slide mechanism 155 which connects the lower plate 26 to the system. This mechanism includes a lower plate slide in a drawer 156 which is attached to the slide mechanism by screws 157.

Turning now to FIG. 3, an opposite side view is further illustrative of the servo valve 40, responsible for proportionally pinching the channels 32. The servo valve 40 is connected by an electrical connector to a circuit board 160, also of conventional design, which controls and interfaces the servo valve 40 with the pressure sensors 44, 45 (FIG. 1) to provide proportional pinching. A power line 162 connects this servo valve 40 to a circuit board also of conventional design (not shown) which regulates power. A bracket 166 supports these servo valves 40.

FIG. 3 also shows the air infusion mechanism 110 including the air fitting 111 and the air tube 114, which receives infusion air from an outside source, extending from the control valve 112 and the air cylinder 118. Two on/off valves 146 are also shown.

FIG. 4 is a top view showing the arrangement of the components on the upper plate 26. This view shows the servo valves 40 with lines 104 leading to the circuit board. The actuator manifold 130 with various valves 132, openings for attaching air tubes 172, and electrical connections 116 are also involved. A bracket 138 supports this manifold 130.

The failsafe valve 150 and its bracket 154 are in view, along with the on/off valves 146, having openings 176 for receiving air tubes from the manifold 130. The air infusion mechanism 110, which includes the pilot valve 122 the cylinder 118 and the control valve 112 are also in this figure.

The upper plate 26 contains pressure sensor openings 180. The pressure sensors 43, 44 (FIG. 1) within these openings include transducers to sense the fluid pressures by pushing against the underlying bag channels 32.

An ejection mechanism 182 for pushing the lower plate 28 out is contained in this device. This allows the lower plate to be removed. Additionally, the infusion unit, as a component of the entire system (not shown) forms a drawer. Screws 184 permanently mount the upper plate 26 to a slide mechanism 156. This slide mechanism includes a grooved member 185 which allows the chilled lower plate 28 to slide in and out. The member 185 also sets the spacing between the chilled lower plate 28 and the upper plate 26. The movement of this bottom plate is monitored by a sensor 186, which assures the lower plate 20 is sufficiently rearward, whereby it will not slide out. This allows the air infusion mechanism to properly activate into the header 34 (FIG. 1).

The invention also contains link pins 188. These link pins 188 maintain the gap between the upper plate 26 and the lower chilled plate 28 when the bag 22 (FIG. 1) fills with infusion fluid. While these link pins 188 are the preferred reinforcements, other suitable reinforcements may be employed.

The preferred embodiment of the infusion device including the infusion bag is relatively small in overall size. Typically the upper and lower plates are about fifteen inches by ten inches, separated by a gap of approximately .050 inches. The bag 22 measures about thirteen inches by eight inches.

The infusion device of the present invention begins operation as an infusion spike on a tube extending from the introduction point attaches to a bottle or other source of pressurized fluid. The fluid moves through the infusion port on the header into the transport channel. The transport channel becomes serpentine in the heat exchange portion, whereby the fluid moving through these serpentine channels becomes chilled, as it is exposed to the cold lower plate for a sufficient time.

The infusion fluid enters either the first flow channel or the second flow channel. Both flow channels are controlled by a servo (proportional) valve, which is controlled with its own pressure sensor. Combined with suitable electronics and logic controls (e.g., computer software), as would be apparent to one skilled in the art, these three components form a servo system to control fluid pressure.

In the servo system, the pressure sensors sense downstream pressure (Po), in the ocular cavity. Acting as feedback devices, the pressure sensors send a signal to the proportional valve which may be pinching at a different pressure (Pv). Upon receiving this signal, a stepper motor in the proportional valve activates and adjusts the proportional pinch up or down based on the difference in pressure $P_\Delta$:

$$P_\Delta = Po - Pv$$

This adjustment is performed to compensate and correct the difference in pressure $P_\Delta$.

For example, a typically requested pressure is 75 mmHg. If the infusion pressure drops to 50 mmHg, due to an decrease in ocular cavity pressure (downstream pressure) caused by any suction, the proportional valve will automatically move to compensate and correct the decrease in pressure by opening to increase the flow rate. Also, typical infusion fluids are prepressurized at 4 psig or about 250 mmHg. This is so that even if all of the valves fail, the ocular cavity will not be damaged at this infusion pressure.

Once the fluid fills the first flow channel, the servo valve adjusts to the proper pressure and fluid may now move to the requested tool at the end of one of three end branch channels. A signal is sent to a manifold valve controlling the corresponding on/off valve to terminate air pressure to this valve, opening the on/off valve for the end branch channel corresponding to the requested tool. These on/off valves can also be activated and deactivated if tool switching is desired.

This process is identical for the second flow channel except that the infusion fluid may only leave through one port, as the port attached to the second end branch channel is closed by the gas/air inlet. This gas/air inlet has been described in operation above. This gas/air inlet normally functions at the end of the surgery.

From the foregoing description, it is clear that those skilled in the art could make changes in the described embodiments and method of the invention without departing from the broad inventive concepts thereof. It is understood, therefore, that this invention is not limited to the particular embodiment disclosed, but it is intended to cover any modifications which are within the spirit and scope of the claims.

## Claims

1. An apparatus for providing fluid under constant pressure for infusion during surgery comprising:
   a bag formed of resilient material having an inlet for receiving said infusion fluid, an outlet attachable to a surgical instrument for carrying said fluid from said bag to a surgical site, said bag having formed therein a channel for conducting said infusion fluid from said inlet to said outlet, said channel having a fluid pressure sensing section and a fluid flow regulating section;
   sensing means contacting said pressure sensing section for sensing pressure of said fluid in said sensing section of said channel and for generating a signal in accordance with sensed pressure; and
   regulating means contacting said flow regulating section for regulating flow of said fluid through said regulating portion of said channel, said regulating means responsive to said sensing means for deforming said regulating section of said channel in accordance with said sensed pressure.

2. The apparatus of claim 1 further comprising:
   a first support plate disposed beneath said bag in contacting relation and for supporting said bag;
   a second support plate disposed above said bag in contacting relation and aligned therewith and having mounted thereon said sensing means and said regulating means.

3. The apparatus of claim 2 wherein:
   said channel further comprises a heat exchange section; and
   said first support plate comprises means contacting said heat exchange section for conducting heat away from said heat exchange section and for cooling said infusion fluid.

4. A disposable bag formed of resilient material for providing fluid under constant pressure for infusion during surgery comprising:
   an inlet for receiving said infusion fluid;
   an outlet attachable to a surgical instrument for carrying said fluid from said bag to a surgical site;
   a channel for conducting said infusion fluid from said inlet to said outlet, said channel having a fluid pressure sensing section and a fluid flow regulating section, said pressure sensing section for contacting a pressure sensor for generating a signal in accordance with fluid pressure in said sensing section, and said regulating section for contacting a flow regulator responsive to said pressure sensor for variably deforming said regulating section in accordance with sensed pressure.

5. A method of manufacturing an infusion bag having a plurality of fluid channels formed therein, and being formed from a pair of thin plastic sheets, comprising the steps of:
   sealing said sheets together in overlying relation substantially completely around their peripheral edges to form a bag, while providing at least one unsealed edge section;

inflating said bag by introducing air into the interior thereof through said at least one unsealed edge section; and

forming said fluid channels in said inflated bag by heat sealing said sheets together along a pair of lines defining each of said channels therebetween.

FIG 1

EP 0 482 858 A2

FIG 2

FIG 3

FIG 4

EP 0 482 858 A2